# EUROPEAN PATENT APPLICATION

(11) **EP 2 957 212 A1**
(43) Date of publication of application: **23.12.2015**
(21) Application number: 14751635.5
(22) Date of filing: 28.01.2014
(51) Int. Cl.: A61B 1/00, G02B 23/24

(54) **RELATIVE POSITION DETECTION SYSTEM FOR TUBE-LIKE DEVICE, AND ENDOSCOPE DEVICE**

(30) Priority: 13.02.2013 JP 2013025395
(71) Applicant: Olympus Corporation, Shibuya-ku Tokyo 151-0072 (JP)
(72) Inventor: FUJITA, Hiromasa, Tokyo 151-0072 (JP); TOJO, Ryo, Tokyo 151-0072 (JP); HANE, Jun, Tokyo 151-0072 (JP); ITO, Takeshi, Tokyo 151-0072 (JP)
(74) Representative: Winter, Brandl, Fürniss, Hübner, Röss, Kaiser, Polte - Partnerschaft mbB
(86) International application number: PCT/JP2014/051778
(87) International publication number: WO 2014/125916

(57) **Abstract**

A relative position detecting system of a tubular device includes a shape sensor (10) and relative position detecting means (25). The shape sensor (10) detects a shape of the tubular device (2) to be inserted into a tubular cavity (4) of an object (3). The relative position detecting means (25) detects a relative positional relation between at least one position in a first range in which the shape of the tubular device (2) is detectable by the shape sensor (10) and at least one position in the tubular cavity (4).

## Description

### Technical Field

The present invention relates to a relative position detecting system which detects positional information or the like of a distal end of a tubular device to be inserted into, for example, a tubular cavity of an object, and an endoscope apparatus to which the relative position detecting system is applied.

### Background Art

For example, in a case where an endoscope or a catheter is inserted into a human body, a pipe or the like as an object, it is convenient that there is information indicating a position of a distal end of the endoscope or the catheter in the human body, the pipe or the like, or information indicating a shape of the inserted endoscope or the catheter.

For example, Jpn. Pat. Appln. KOKAI Publication No. S60-217326 (PTL 1) discloses, to detect a position of an endoscope distal end to an object, bend angle information output means for outputting information corresponding to a bend angle of the endoscope distal end rotation angle detecting means for arranging an inlet guide member disposed in an object insertion port and detecting a relative rotation angle of the endoscope with the inlet guide member, insertion length detecting means for detecting a length of the endoscope inserted into the object, and means for displaying an object inside schematic image and an endoscope simulation image to be positioned in accordance with the information corresponding to the bend angle and insertion length information. PTL 1 discloses, as another method of detecting the insertion length of the endoscope, a method in which an ultrasonic receiver is disposed in a head of the object and ultrasonic waves emitted from the endoscope distal end are detected to detect the insertion length of the endoscope.

### Citation List

### Patent Literature

PTL 1: Jpn. Pat. Appln. KOKAI Publication No. S60-217326

### Summary of Invention

### Technical Problem

In PTL 1, to detect an insertion length or a rotation angle of an endoscope to an object, an inlet guide member is disposed in an object insertion port, and in this inlet guide member, there are provided rotation angle detecting means for detecting a relative rotation angle of the endoscope and insertion length detecting means for detecting a length of the endoscope inserted in the object. Thus, the inlet guide member can be disposed at any position as long as a part of the inlet guide member is inserted into the insertion port of the object, e.g., a mouth part of a human body and is easily fixed and held.

However, PTL 1 has the following problem. There is a region where the inlet guide member is hard to be fixed and held in the insertion port of the object. When an operator operates the endoscope, the inlet guide member becomes a disturbance, if any. It requires a great deal of time and labor to fix the inlet guide member. The inlet guide member needs to be washable or disposable, which requires much cost. When an insertion angle of the endoscope to the object cannot be kept to be vertical, an error is included in a calculation result. When an object inside schematic image does not have an accurate shape, a distal end position cannot accurately be calculated. When the object inside schematic image does not have the accurate shape, the insertion length cannot be calculated. When the inside of the object is a moving region or structure, the insertion length cannot be calculated. Furthermore, for example, although an operation of inserting the endoscope into a large intestine of the human body is performed, there occurs a situation where a distal end of the endoscope does not move forward in the large intestine in a certain case. In this case, when an insertion amount of the endoscope is only detected, it cannot be distinguished whether the distal end of the endoscope moves forward in the large intestine, or whether the distal end of the endoscope does not move forward but the endoscope is inserted in the large intestine.

An object of the present invention is to provide a relative position detecting system of a tubular device which is capable of accurately acquiring an arrangement of the tubular device to a tubular cavity, and an endoscope apparatus.

### Solution to Problem

A relative position detecting system of a tubular device according to an aspect of the invention includes a shape sensor for detecting a shape of the tubular device to be inserted into a tubular cavity of an object, and relative position detecting means for detecting a relative positional relation between at least one position in a first range in which the shape of the tubular device is detectable by the shape sensor and at least one position in the tubular cavity.

An endoscope apparatus according to an aspect of the invention includes a shape sensor for detecting a shape of the tubular device to be inserted into a tubular cavity of an object, relative position detecting means for detecting a relative positional relation between at least one position in a first range in which the shape of the tubular device is detectable by the shape sensor and at least one position in the tubular cavity, and an operating portion that operates a shape of the inserting portion.

### Advantageous Effects of Invention

According to the present invention, it is possible to provide a relative position detecting system of a tubular device which is capable of accurately acquiring an arrangement of the tubular device to a tubular cavity, and an endoscope apparatus.

### Brief Description of Drawings

FIG. 1 is a functional block diagram in which an embodiment of a relative position detecting system according to the present invention is applied to an endoscope apparatus;
FIG. 2 is an external configuration diagram showing the relative position detecting system;
FIG. 3 is a configuration diagram showing a light emission detecting device in the relative position detecting system;
FIG. 4A is a schematic diagram showing that a light transmission quantity is large in accordance with a bend of a shape sensor in the relative position detecting system;
FIG. 4B is a schematic diagram showing that the light transmission quantity is medium in accordance with the bend of the shape sensor in the relative position detecting system;
FIG. 4C is a schematic diagram showing that the light transmission quantity is small in accordance with the bend of the shape sensor in the relative position detecting system;
FIG. 5 is a schematic diagram showing a detecting operation of a positional relation by a relative position detecting section in the relative position detecting system;
FIG. 6 is a schematic diagram showing first and second detecting elements of the relative position detecting section in the relative position detecting system;
FIG. 7 is a schematic diagram showing the first and second detecting elements that are present in a common coordinate system in the relative position detecting system;
FIG. 8 is a schematic diagram when a magnetic sensor is used in the first detecting element of the relative position detecting system;
FIG. 9 is a schematic diagram when an acceleration sensor is used in the first detecting element of the relative position detecting system;
FIG. 10 is a diagram showing a calculating operation of positional information when the acceleration sensor is used in the first detecting element of the relative position detecting system; and
FIG. 11 is a schematic diagram when an acceleration sensor is used in the second detecting element of the relative position detecting system.

### Description of Embodiments

Hereinafter, one embodiment of the present invention will be described with reference to the drawings.

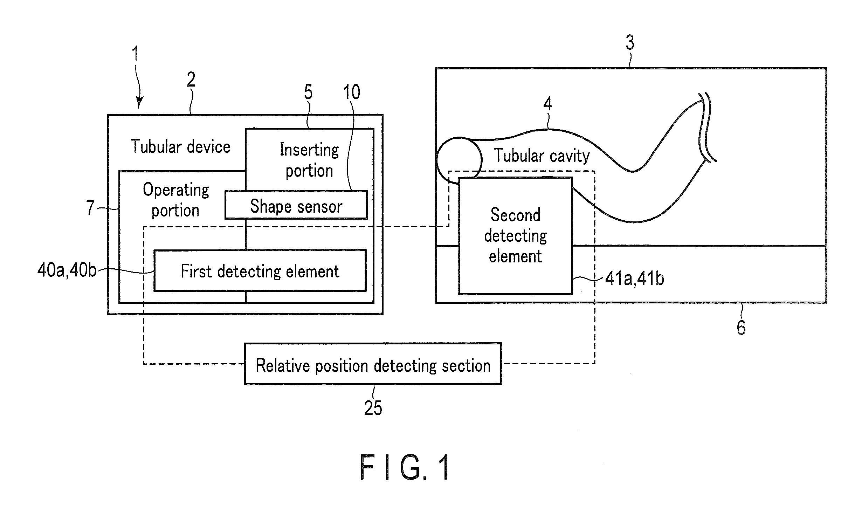

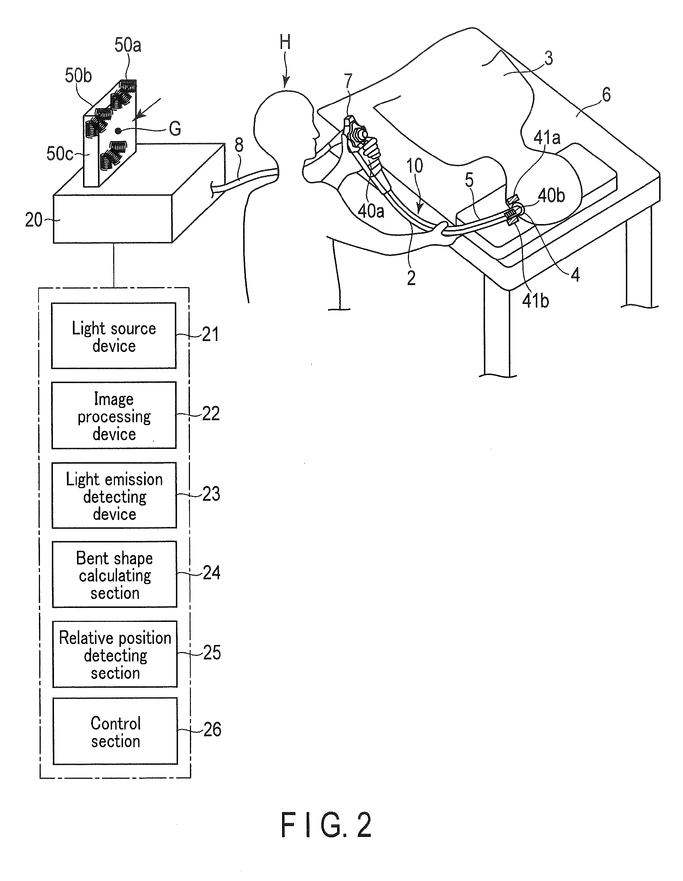

FIG. 1 shows a functional block diagram of an endoscope apparatus to which a relative position detecting system is applied, and FIG. 2 shows an external configuration diagram of the endoscope apparatus. H shown in FIG. 2 is an operator of the endoscope apparatus who is a surgeon or the like.

An endoscope apparatus 1 includes a hollow and elongate tubular device 2. In the endoscope apparatus 1, the tubular device 2 is inserted into a tubular cavity 4 of an object 3 that is, e.g., a human body of a patient or the like, and an image of an inside of the tubular cavity 4 is acquired, to preform, e.g., observation or treatment of a lesion part or the like in the tubular cavity 4, sampling of, e.g., the lesion part or foreign substances, or the like.

An inserting portion 5 made of a flexible material is formed on a distal end side of the tubular device 2. The inserting portion 5 is inserted into the tubular cavity 4 of the object 3. The inserting portion 5 bends in an upward-downward direction (UD direction) and a right-left direction (RL direction), and is actually inserted into the tubular cavity 4. At a distal end of the inserting portion 5, an imaging device is disposed, and this imaging device acquires an image of the inside of the tubular cavity 4 to output an image signal thereof. The tubular cavity 4 is a body cavity (a lumen) of the human body, e.g., a region from a mouth part to an organ such as an esophagus or a stomach part, or a region from an anus to an organ such as a large intestine. The object 3 is mounted on, e.g., an inspection table 6.

In the endoscope apparatus 1, an operating portion 7 to operate the tubular device 2 is disposed. The operating portion 7 is coupled with a proximal end of the tubular device 2. The operating portion 7 is configured to perform an operation of bending the inserting portion 5 in the UD direction and the RL direction, and includes an UD angle knob to perform the operation in the UD direction and an RL angle knob to perform the operation in the RL direction. For example, when the observation, the treatment or the like of the lesion part is performed, an operator operates the UD angle knob and the RL angle knob to bend the inserting portion 5 in the UD direction and the RL direction. In the operating portion 7, there are provided various switches and the like to perform functions mounted in the inserting portion 5, e.g., imaging, scavenging, cleaning of the distal end of the inserting portion 5, and liquid supplying.

In the tubular device 2, a shape sensor 10 is disposed. The shape sensor 10 is configured to detect a shape of the tubular device 2, and includes, e.g., an optical fiber sensor.

The shape sensor 10 is disposed to the tubular device 2 so that the shape of the tubular device 2 is transmitted to the shape sensor using at least the inserting portion 5 of the tubular device 2 as a sensitive material.

The shape sensor 10 detects optical information corresponding to a direction and a size of a bend as a shape of the inserting portion 5 in the tubular device 2 when the inserting portion 5 bends.

The tubular device 2 is connected to a tubular device processing apparatus 20 via a universal cord 8. The tubular device processing apparatus 20 inputs the image signal output from the imaging device of the tubular device 2, processes this image signal to acquire an image of the inside of the tubular cavity 4 (hereinafter referred to as an endoscope image), and displays this endoscope image in, e.g., a display. The tubular device processing apparatus 20 includes a light source device 21, an image processing device 22, a light emission detecting device 23, a bent shape calculating section 24, a relative position detecting section 25, and a control section 26.

The light source device 21 emits illumination light toward a distal end of the tubular device 2, i.e., the distal end of the inserting portion 5 via the universal cord 8. At the distal end of the inserting portion 5, a light emission port is disposed, and the illumination light is emitted from this light emission port to irradiate the inside of the tubular cavity 4 of the object 3. Furthermore, the imaging device disposed at the distal end of the inserting portion 5 images reflected light from the inside of the tubular cavity 4 of the object 3, and outputs the image signal thereof. This image signal is sent to the tubular device processing apparatus 20 via the universal cord 8.

The image processing device 22 inputs the image signal output from the imaging device, and processes this image signal to acquire the endoscope image of the inside of the tubular cavity 4 of the object 3, thereby displaying the endoscope image in, e.g., the display as described above.

The light emission detecting device 23 receives the optical information corresponding to the direction and size of the bend which is output from the shape sensor 10 when the inserting portion 5 bends, e.g., a light transmission quantity of the shape sensor 10, and detects the direction and the size of the bend when the inserting portion 5 bends.

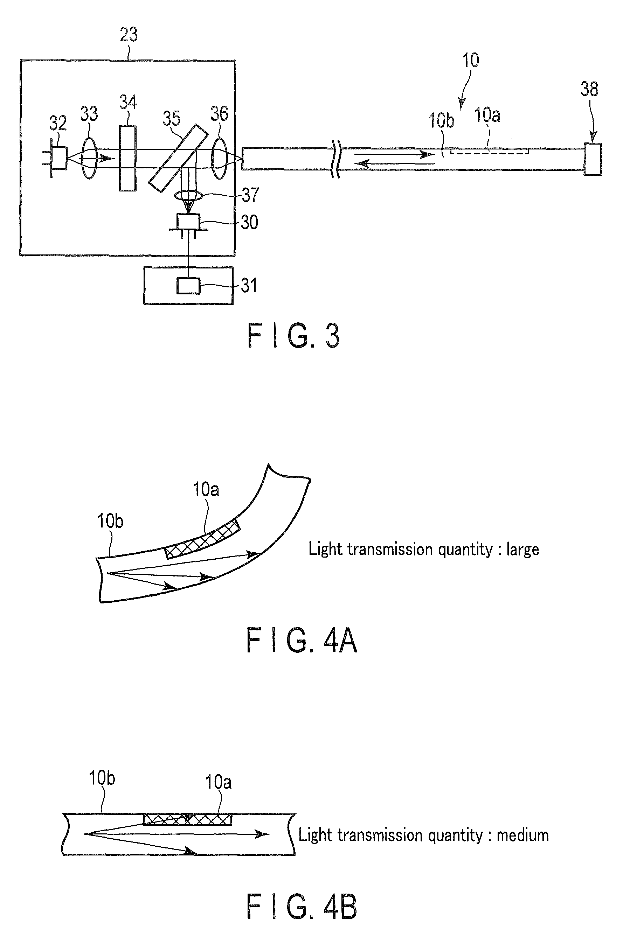

FIG. 3 shows a configuration diagram of the light emission detecting device 23. As described above, the shape sensor 10 includes, e.g., the optical fiber sensor. In the shape sensor 10, when the inserting portion 5 bends, an optical fiber 10b constituting the optical fiber sensor bends in accordance with this bend, and part of the light to be accordingly transmitted through the optical fiber 10b exits (leaks) to the outside through a light detecting portion 10a. That is, the light detecting portion 10a is disposed in one side surface of the optical fiber 10b, and emits, to the outside, part of the light to be transmitted in accordance with the bend of the optical fiber 10b. Namely, the light detecting portion 10a changes optical characteristics, e.g., the light transmission quantity of the optical fiber 10b.

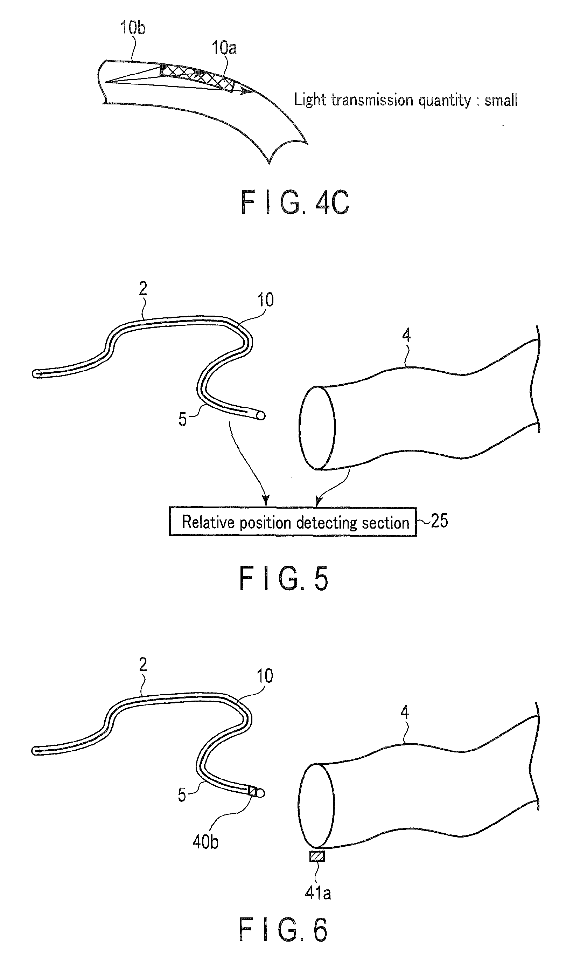

FIG. 4A, FIG. 4B and FIG. 4C show schematic diagrams of the light transmission quantity in accordance with the bend of the optical fiber 10b. FIG. 4A shows the light transmission quantity when the optical fiber 10b bends to a side on which the light detecting portion 10a is disposed. FIG. 4B shows the light transmission quantity when the optical fiber 10b does not bend. FIG. 4C shows the light transmission quantity when the optical fiber 10b bends to a side opposite to the side on which the light detecting portion 10a is disposed. As shown in FIG. 4A, FIG. 4B and FIG. 4C, the light transmission quantity when the optical fiber 10b bends to the side on which the light detecting portion 10a is disposed is largest. The light transmission quantity when the optical fiber 10b does not bend is large next. The light transmission quantity when the optical fiber 10b bends to the side opposite to the side on which the light detecting portion 10a is disposed is large next.

Specifically, the light emission detecting device 23 includes a light source 32 for the optical fiber sensor. The light emission detecting device 23 is configured by arranging a projection lens 33, an isolator 34, a reflecting mirror 35, and a condensing lens 36 on a light path of the light emitted from the light source 32. On a light path of the light to be condensed by the condensing lens 36, the optical fiber 10b is disposed.

On a reflected light path of the reflecting mirror 35, a bent shape detecting section 30 is disposed via a condensing lens 37. An output signal of the bent shape detecting section 30 is sent to the bent shape calculating section 24. The light source 32 includes, for example, a laser diode (LD).

The projection lens 33 projects the light emitted from the light source 32.

The condensing lens 36 condenses the light on the optical fiber 10b so that the light passing through the isolator 34 and the reflecting mirror 35 enters the optical fiber 10b.

The reflecting mirror 35 emits, to the condensing lens 30, 36, the light emitted from the light source 32 and transmitted through the isolator 34, and further, reflects, toward the condensing lens 37, the light that is emitted from the optical fiber 10b to return through the condensing lens 36.

At a distal end of the optical fiber 10b, a reflecting mirror 38 is disposed. The reflecting mirror 38 is configured to return the light that has entered the optical fiber 10b.

When the light detecting portion 10a changes the optical characteristics, the bent shape detecting section 30 detects a bent shape of the optical fiber 10b, specifically the direction and size of the bend on the basis of these changed optical characteristics, e.g., the light transmission quantity.

A bent shape calculating section 31 calculates a bent shape of an actually bent portion on the basis of the detection result of the bent shape detecting section 30. The optical characteristics are not limited to, e.g., the light transmission quantity, and may be a state of the light, e.g., spectrum or polarization. The bent shape detecting section 30 may detect the optical characteristics corresponding to the light quantity or the light state, e.g., the spectrum or the polarization. The relative position detecting section 25 detects a relative positional relation between at least one position in the tubular device 2 and at least one position in the tubular cavity 4. FIG. 5 shows a schematic diagram of a detecting operation of the positional relation by the relative position detecting section 25.

Here, a range in which the whole shape of the tubular device 2 can be detected by the shape sensor 10 is defined as a first range. A range in which a relative position to a range of the inserting portion 5 in the tubular device 2 can be specified is defined as a second range. The relative position detecting section 25 detects a relative positional relation between at least one position in the first range in which the shape can be detected by the shape sensor 10 in the tubular device 2 and at least one position on the tubular cavity 4.

The relative position detecting section 25 detects a relative positional relation between at least one position in one of the first range in which the shape can be detected by the shape sensor 10 and the second range in which the relative position to the range of the inserting portion 5 in the tubular device 2 can be specified and at least one position on the tubular cavity 4.

The relative position detecting section 25 acquires arrangement information of the tubular device 2 to the tubular cavity 4 on the basis of the shape of the tubular device 2 which is detected by the shape sensor 10, at least one position of the inserting portion 5, and at least one position on the tubular cavity 4. The arrangement information of the tubular device 2 includes positional information of the distal end of the inserting portion 5 in the tubular device 2.

Specifically, the relative position detecting section 25 includes at least first detecting elements 40a, 40b that are present in at least one position of the inserting portion 5 as shown in FIG. 2 in one of the first range and the second range, includes at least second detecting elements 41a, 41b that are present at the object 3, and detects relative positions of the first detecting elements 40a, 40b and the second detecting elements 41a, 41b. FIG. 6 shows a schematic diagram of an example where the first detecting element 40b is disposed at the distal end of the inserting portion 5 in the tubular device 2 and the second detecting element 41a is disposed in a periphery of a mouth part of the object 3.

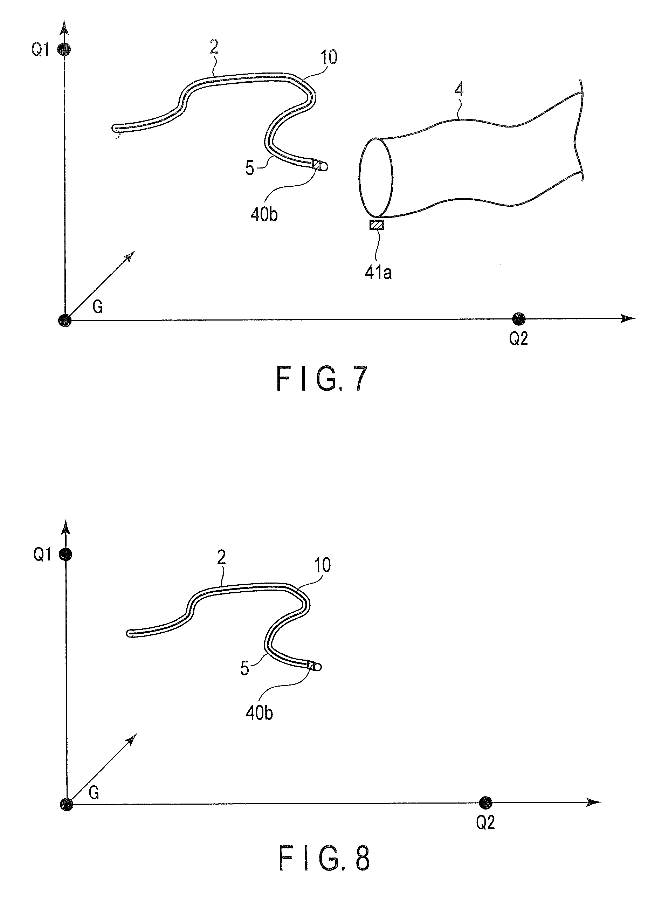

The first detecting elements 40a, 40b and the second detecting elements 41a, 41b are respectively present in a common coordinate system. FIG. 7 shows a schematic diagram of the first detecting elements 40a, 40b and the second detecting elements 41a, 41b that are present in the common coordinate system. Each of the first detecting elements 40a, 40b includes a first transmitter or a first receiver that is fixed and disposed, and each of the second detecting elements 41a, 41b includes a second receiver or a second transmitter that is configured to be able to detect its relative position to the first transmitter or the first receiver. In each of, e.g., three positions G, Q1 and Q2 including the origin G of the common coordinate system, a third receiver or a third transmitter is disposed.

Between the first transmitter and the second receiver, information is transmitted and received in accordance with a signal including a magnetic field, an electromagnetic wave, a sound wave or an ultrasonic wave. Also between the second transmitter and the first receiver, information is transmitted and received in accordance with a signal including a magnetic field, an electromagnetic wave, a sound wave or an ultrasonic wave. For example, in a case where the first detecting element 40a is the first transmitter and the second detecting element 41a is the second receiver, when the first detecting element 40a transmits, e.g., the magnetic field, the second detecting element 41a receives the magnetic field from the first detecting element 40a.

Specifically, as shown in FIG. 8, the first detecting element 40b detects a position in a space including a plane between the position and a predetermined point such as the origin G shown in FIG. 2, and includes a magnetic sensor or the like. In the first detecting element 40b, the magnetic sensor constitutes the transmitter or the receiver. The origin G is set to, e.g., an arrangement position of the tubular device processing apparatus 20. At the origin G, for example, coils 50a, 50b and 50c are arranged as position detecting elements as shown in FIG. 2. The coils 50a, 50b and 50c generate, for example, a magnetic field to detect the first detecting elements 40a, 40b, and receive a magnetic field generated in the first detecting elements 40a, 40b by inducing by the magnetic field generated by the coils 50a, 50b and 50c. The tubular device processing apparatus 20 obtains absolute positions of the first detecting elements 40a, 40b by use of the origin G as a reference, on the basis of a size or the like of the magnetic field received by each of the coils 50a, 50b and 50c.

The first detecting element 40a or 40b is mounted in at least one position on the tubular device 2 in one of the first range in which the whole shape of the tubular device 2 can be detected by the shape sensor 10 and the second range in which the relative position to the range of the inserting portion 5 in the tubular device 2 can be specified.

The first detecting elements 40a, 40b are disposed in an inlet region of the tubular cavity 4 to acquire information including the arrangement of the tubular device 2 on the basis of a predetermined position such as the origin G, e.g., a mouth part or an anus, and in an inner region of the tubular cavity 4, e.g., an organ such as a large intestine.

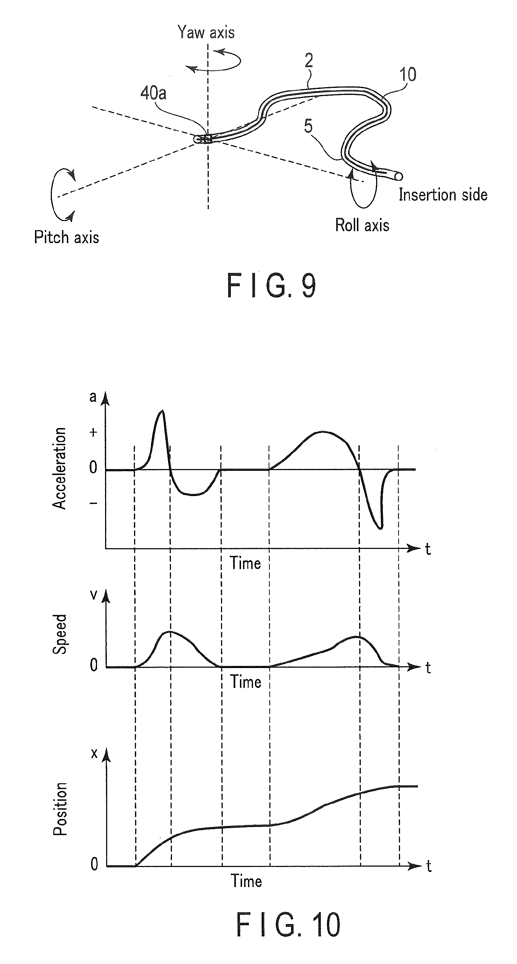

As shown in FIG. 9, the first detecting element 40a is one or both of an acceleration sensor that detects an acceleration to be applied to the tubular device 2 and a rotation sensor that detects at least a rotation amount of the tubular device 2. The first detecting element 40a is mounted in at least one position on the tubular device 2 in one of the first range in which the whole shape of the tubular device 2 can be detected by the shape sensor 10 and the second range in which the relative position to the range of the inserting portion 5 in the tubular device 2 can be specified. Here, the first detecting element 40a is disposed at a proximal end of the inserting portion 5 in the tubular device 2.

When the first detecting element 40a is the acceleration sensor, the acceleration sensor detects the acceleration to be applied to the proximal end of the inserting portion 5 in the tubular device 2, and outputs a signal of the acceleration as shown in FIG. 10. This acceleration signal is sent to, e.g., the tubular device processing apparatus 20. The tubular device processing apparatus 20 integrates the acceleration signal once to obtain a speed of the proximal end of the inserting portion 5, and further, performs the integration (second integration) to obtain positional information as shown in FIG. 10.

When the first detecting element 40a is the rotation sensor, this rotation sensor detects an inclination and the rotation amount of the proximal end of the inserting portion 5 in the tubular device 2, e.g., the inclination of each of a yaw axis, a pitch axis and a roll axis and the rotation amount around the each axis.

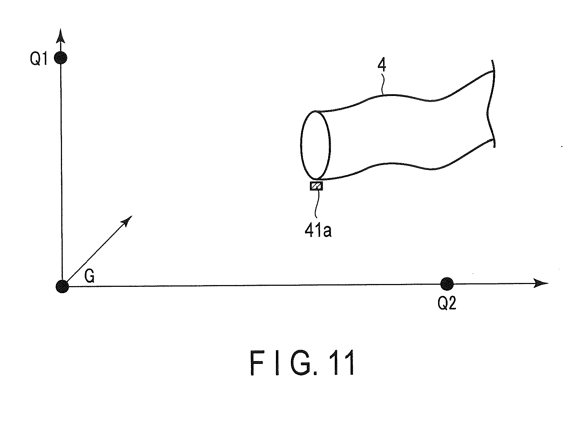

As shown in FIG. 11, the second detecting elements 41a, 41b include a magnetic sensor. For example, the second detecting element 41a is disposed in at least one position of the tubular cavity 4, e.g., the periphery of the mouth part of the object 3.

The second detecting element 41a or 41b is disposed at a position at which a positional relation between the position and the tubular cavity 4 is specified, e.g., the inspection table 6 on which the object 3 is mounted, and detects positional information of an inlet of the tubular cavity 4. For example, at each of three positions G, Q1 and Q2 including the origin G of the coordinate system, an antenna or a transmitter is disposed.

The control section 26 controls each of the light source device 21, the image processing device 22, the light emission detecting device 23, the bent shape calculating section 24 and the relative position detecting section 25 in the tubular device processing apparatus 20, and performs a series of operation control to input the image signal output from the imaging device of the tubular device 2, process this image signal to acquire the endoscope image of the inside of the tubular cavity 4, and display this endoscope image in, e.g., the display.

Next, an operation effect of the relative position detecting system constituted as described above will be described.
(1) As shown in FIG. 2, the operator H, e.g., the surgeon operates the endoscope apparatus to dispose the inserting portion 5 of the tubular device 2 at a predetermined position, e.g., a position that requires positional information or arrangement information of the tubular device 2 from the inlet of the tubular cavity 4, i.e., the mouth part of the patient or the like, a position of an inlet of an organ in the tubular cavity 4, a separating region of a blood vessel, or the like, and further, inserts the inserting portion from the mouth part of the patient or the like into the tubular cavity 4 of a stomach part or the like. The endoscope apparatus 1 starts imaging, performs the imaging from the inlet of the tubular cavity 4 to the inside of the tubular cavity 4 of the stomach part or the like to acquire the images, and performs the observation or treatment of the lesion part or the like in the tubular cavity 4, the sampling of, e.g., the lesion part or the foreign substances, or the like. When the inserting portion 5 of the tubular device 2 is inserted from, e.g., the mouth part of the patient into the tubular cavity 4 of the stomach part or the like, the inserting portion bends into a shape following the shape of the tubular cavity 4 to move forward in the tubular cavity 4.

Here, when the inserting portion 5 of the tubular device 2 is disposed at the predetermined position, e.g., the inlet of the tubular cavity 4, the shape sensor 10 detects the optical information corresponding to the direction and the size of the bend when the inserting portion 5 bends, as the shape of the inserting portion 5 in the tubular device 2. That is, as shown in FIG. 3, when the light is emitted from the light source 32 in the light emission detecting device 23, this light enters the optical fiber 10b through the projection lens 33, the isolator 34, the reflecting mirror 35, and the condensing lens 36. The light that has entered the optical fiber 10b propagates in the optical fiber 10b, and is reflected by the reflecting mirror 38 to return in the optical fiber 10b.

At this time, when the inserting portion 5 bends, the optical fiber 10b of the shape sensor 10 which constitutes the optical fiber sensor bends in accordance with the bend of the inserting portion 5, and accordingly, part of the light transmitted in the optical fiber 10b exits (leaks) to the outside through the light detecting portion 10a. That is, the light detecting portion 10a changes the optical characteristics of the optical fiber 10b, e.g., the light transmission quantity. As to this light transmission quantity, as shown in FIG. 4A, FIG. 4B and FIG. 4C, the light transmission quantity when the optical fiber 10b bends to the side on which the light detecting portion 10a is disposed is largest, the light transmission quantity when the optical fiber 10b does not bend is large next, and the light transmission quantity when the optical fiber 10b bends to the side opposite to the side on which the light detecting portion 10a is disposed is large next. The light of such a light transmission quantity is reflected by the reflecting mirror 35, and enters the bent shape detecting section 30 via the condensing lens 37. The bent shape detecting section 30 sends, to the bent shape calculating section 24, the signal corresponding to the light transmission quantity of the light that has entered.

The bent shape calculating section 24 calculates the bent shape of the actually bent portion on the basis of the light transmission quantity of the light which is the detection result of the bent shape detecting section 30.

The relative position detecting section 25 acquires the arrangement information of the tubular device 2 to the tubular cavity 4 on the basis of the shape of the tubular device 2 which is detected by the shape sensor 10 to be obtained by the bent shape calculating section 24, at least one position of the inserting portion 5, and at least one position on the tubular cavity 4.
(2) As shown in FIG. 5, the relative position detecting section 25 detects the relative positional relation between at least one position in the first range in which the shape can be detected by the shape sensor 10 in the tubular device 2 and at least one position on the tubular cavity 4.

Specifically, the relative position detecting section 25 detects the relative positional relation between at least one position in one of the first range in which the shape can be detected by the shape sensor 10 and the second range in which the relative position to the range of the inserting portion 5 in the tubular device 2 can be specified and at least one position on the tubular cavity 4.

Specifically, the relative position detecting section 25 acquires the arrangement information of the tubular device 2 to the tubular cavity 4 on the basis of the shape of the tubular device 2 which is detected by the shape sensor 10, i.e., the bent shape of the tubular device 2 which is obtained by the bent shape calculating section 24, at least one position of the inserting portion 5, and at least one position on the tubular cavity 4. The arrangement information of the tubular device 2 includes the positional information of the distal end of the inserting portion 5 in the tubular device 2.

Consequently, the relative positional relation between at least one position in the first range in which the shape can be detected by the shape sensor 10 in the tubular device 2 and at least one position on the tubular cavity 4 is detected, and hence, the arrangement of the tubular device 2 to the tubular cavity 4 can accurately be acquired. That is, when there is information indicating a specific region of the human body, a pipe or the like in which a distal end of the endoscope apparatus 1 or a catheter is present and a specific shape in which the distal end is inserted, it is convenient. For example, the catheter is irradiated with an X-ray to detect a distal end position of the catheter, but when the information of the distal end position of the catheter or the shape of the catheter is present as described above, the current distal end position or a current direction in which the catheter moves forward is known without irradiating the catheter with the X-ray, so that an irradiation quantity of the X-ray can be decreased.

In the endoscope apparatus 1, a place that is being observed can be specified, and a specific position of a middle of a scope of the inserting portion 5 or the like that is deformed and bends or loops can be understood. Therefore, it is possible to provide the relative position detecting system of the tubular device which can be used in education of the operator H or can safely and easily be used even by the operator H who is not accustomed to the operation.

In the present system, even at one point of the first range in which the whole shape of the tubular device 2 including the inserting portion 5 of, e.g., the catheter incorporating the shape sensor 10 can be detected by the shape sensor 10 or the second range in which the relative position to the range of the inserting portion 5 can be specified, the relative position detecting section 25 that detects the relative position to the tubular cavity 4 is disposed, and hence, at least one piece of positional information of the tubular device 2 to the tubular cavity 4 can be acquired. The shape sensor 10 acquires the shape of the tubular device 2 on the basis of such positional information, and hence, the whole arrangement information of the tubular device 2 to the tubular cavity 4 can be acquired.
(3) As shown in FIG. 6, the relative position detecting section 25 includes at least the first detecting element 40b that is present in at least one position of the inserting portion 5 in one of the first range in which the whole shape of the tubular device 2 can be detected by the shape sensor 10 and the second range in which the relative position to the range of the inserting portion 5 in the tubular device 2 can be specified, and the second detecting element 41a that is present at the object 3, and detects the relative position of the first detecting element 40b to the second detecting element 41a.

In consequence, the tubular device processing apparatus 20 can acquire at least one piece of positional information of the tubular device 2 by the first detecting element 40b. Thus, the whole shape of the tubular device 2 is acquired by the shape sensor 10 on the basis of at least one piece of positional information of the tubular device 2, and hence, it is possible to acquire the whole arrangement information of the tubular device 2 to at least one piece of positional information of the tubular device 2.

The tubular device processing apparatus 20 can acquire the positional information of the object 3 by the second detecting element 41a, and obtain the position of the tubular cavity 4 to the object 3 from the arrangement information of the tubular cavity 4.

The tubular device processing apparatus 20 combines the whole shape of the tubular device 2 and the relative position of the tubular device to the positional information of the object 3, so that it is possible to acquire the whole arrangement information of the tubular device 2 to the tubular cavity 4 in the object 3.
(4) In the shape sensor 10, as shown in FIG. 3, the optical fiber sensor including the optical fiber 10b is used to detect the bent shape (a bending amount) of the inserting portion 5. This optical fiber sensor can be constituted in a sufficiently thin shape to the tubular device 2, can therefore easily be mounted in the tubular device 2, and is hard to be influenced by another constitution.

As the optical fiber sensor, there is utilized one example where the light detecting portion 10a that absorbs the light is disposed at a predetermined position of the optical fiber 10b as shown in FIG. 3. The light detecting portion 10a detects the bending amount and a bending direction due to decrease or increase of a quantity of the light propagating through a core of the optical fiber 10b when the optical fiber bends in a predetermined direction. For example, the light transmission quantity is largest when the optical fiber 10b bends to the side on which the light detecting portion 10a is disposed as shown in FIG. 4A, and next, the light transmission quantity when the optical fiber 10b does not bend as shown in FIG. 4B and next, the light transmission quantity when the optical fiber 10b bends to the side opposite to the side on which the light detecting portion 10a is disposed as shown in FIG. 4C are large in this order. Any optical fiber sensor having such a constitution can inexpensively be constituted.

This optical fiber sensor outputs a signal corresponding to the bending amount at a distance at which the bent shape of the tubular device 2 can be detected. The bent shape calculating section 24 calculates and obtains the bent shape or the bending amount of the tubular device 2 on the basis of the signal output from the optical fiber sensor. The bent shape calculating section 24 estimates a partial shape of the tubular device 2 which changes together with the bending amount. Finally, the bent shape calculating section 24 joins these partial shapes together to calculate the whole shape of the tubular device 2.

The bent shape calculating section 24 preferably calculates and obtains the shape of the tubular device 2 on the basis of, e.g., the position of the first detecting element 40b. The first detecting elements 40a, 40b and the like are disposed at positions, so that the shape of the tubular device 2 can more accurately be obtained. In another calculation of the shape of the tubular device 2, a refractive index in the optical fiber 10b changes together with the bend, and hence, a wavelength shift amount of reflected light is detected from this refractive index (FBG), or a place is specified in combination with return time of the light to detect bending amounts at the positions. Thus, any system may be used as long as similar operation and effect can be obtained.
(5) As shown in FIG. 7, the first detecting elements 40a, 40b and the second detecting elements 41a, 41b are present in the common coordinate system, and include the first transmitter or the first receiver that is fixed and disposed, and the second receiver or the second transmitter that is configured to be able to detect the relative position to the first transmitter or the first receiver, respectively. In consequence, the relative position detecting section 25 obtains respective coordinates of the first detecting elements 40a, 40b and the second detecting elements 41a, 41b in the common coordinate system based on a predetermined position such as the origin G, so that the relative positional relation between at least one position in the tubular device 2 and at least one position on the tubular cavity 4 can easily be detected.
(6) In the first detecting elements 40a, 40b and the second detecting elements 41a, 41b, the signals transmitted and received between the first transmitter and the second receiver and between the second transmitter and the first receiver include magnetic fields, electromagnetic waves, sound waves or ultrasonic waves, respectively. Consequently, in communication among the position of the origin G as a reference, each of the first detecting elements 40a, 40b and each of the second detecting elements 41a, 41b, a radio signal is transmitted and received without requiring any wires, so that it is possible to detect the relative positional relation between at least one position in the tubular device 2 and at least one position on the tubular cavity 4. In the transmission/reception of the signal by radio, the operator H who operates the tubular device 2 is not disturbed. In addition, the radio signal other than the sound wave does not disturb the operator H or the object 3 because the signal is not felt.
(7) As shown in FIG. 8, each of the first detecting elements 40a, 40b is the magnetic sensor that detects a position in the space including the plane between the position and the predetermined position. Each of the first detecting elements 40a, 40b is mounted in at least one position on the tubular device 2 in one of the first range in which the shape can be detected by the shape sensor 10 and the second range in which the relative position to the range of the inserting portion 5 in the tubular device 2 can be specified.

Consequently, in the relative position detecting section 25, the output signals of the magnetic sensor of the first detecting element 40a or 40b or the shape sensor 10 are successively calculated from a time when the tubular device 2 is disposed at the predetermined position, to easily detect the relative positional relation between at least one position in the tubular device 2 and at least one position on the tubular cavity 4. Consequently, in a state where any member that disturbs the insertion of the tubular device 2 is not present at the inlet of the tubular cavity 4 of the object 3 and the tubular device 2 is easily inserted, it is possible to obtain the positional information of the tubular device to the tubular cavity 4 and the arrangement information of the tubular device 2.

The predetermined position is, for example, a region that requires the positional information of the tubular device 2 and the arrangement information of the whole tubular device from that position, such as the inlet of the tubular cavity 4, the inlet of the organ in the tubular cavity 4, or the separating region of the blood vessel. When the first detecting element 40b is disposed close to an insertion end of the inserting portion 5 of the tubular device 2, shape calculation is performed from a starting point at which the first detecting element 40b is disposed, so that it is possible to more accurately detect the position of the distal end of the tubular device 2.

At the origin G, as shown in FIG. 2, the coils 50a, 50b and 50c are disposed as the position detecting elements, and generate the magnetic field signals to the first detecting elements 40a, 40b or receive the magnetic fields generated from the first detecting elements 40a, 40b. In consequence, the operator H who operates the tubular device 2 can obtain an absolute position based on the predetermined position without worrying about the wires or the like.

The positional relation between the first detecting element 40a or 40b and the shape sensor 10 is specified, and hence, it is possible to detect an arrangement state of the tubular device 2 in the space on the basis of the first detecting element 40a or 40b.

The first detecting element 40a or 40b includes a constitution capable of detecting a strength and a direction of the magnetic field between the detecting element and each of two or more fixed receivers or transmitters. For example, a coil (a longitudinal side coil) is disposed in one direction along a longitudinal axis of the scope in the tubular device 2. At two fixed points, coils (fixed side coils) are disposed toward a biaxial or triaxial direction.

A current is allowed to flow in the longitudinal side coil to generate the magnetic field, the fixed side coils receive the magnetic field, and the position and direction of coil disposed in the tubular device 2 are calculated and obtained from each of differences between magnetic field strengths of these two points and between electric field strengths of the coils in the biaxial or triaxial direction. As to a space between the receivers at the two or more fixed points, the receivers are disposed via a predetermined distance in accordance with a necessary resolution of the position. The coil provided in the tubular device 2 is disposed as close to an insertion end side of the tubular device 2 as possible. In consequence, it is possible to more accurately obtain the positions and arrangement of the tubular device 2 and the tubular cavity 4. The coil is not limited to one position of the tubular device 2, and the coils may be disposed at positions in the tubular device.
(8) As shown in FIG. 9, the first detecting element 40a includes one or both of the acceleration sensor that detects the acceleration to be applied to the tubular device 2 and the rotation sensor that detects at least the rotation amount of the tubular device 2, and the first detecting element is mounted in at least one position on the tubular device 2 in one of the first range in which the shape can be detected by the shape sensor 10 and the second range in which the relative position to the range of the inserting portion 5 in the tubular device 2 can be specified.

When the inserting portion 5 of the tubular device 2 is disposed at a predetermined position such as the inlet of the tubular cavity 4, the first detecting element 40a starts acquiring at least one piece of positional information of the tubular device 2. When the first detecting element 40a is the acceleration sensor, the acceleration sensor detects the acceleration to be applied to the proximal end of the inserting portion 5 in the tubular device 2 as shown in FIG. 10, and outputs the acceleration signal. As shown in FIG. 10, the tubular device processing apparatus 20 integrates the acceleration signal once to obtain the speed of the proximal end of the inserting portion 5, and further, performs integration (the second integration) to obtain the positional information.

When the first detecting element 40a is the rotation sensor, the rotation sensor detects the inclination of the proximal end of the inserting portion 5 in the tubular device 2 and the rotation amount of the proximal end, e.g., the inclination of the proximal end along each of the yaw axis, the pitch axis and the roll axis and the rotation amount around the each axis.

The acceleration sensor or the rotation sensor is disposed in the range in which the relative position of the sensor to the shape sensor 10 can be specified, and hence, an insertion amount of the tubular device 2 into the tubular cavity 4, the rotation amount of the tubular device 2 and the shape of the tubular device 2 are combined, so that it is possible to obtain the relative position of the tubular device 2 in the tubular cavity 4 and the arrangement information of the tubular device.

The range in which the relative position to the range of the inserting portion 5 can be specified is a range that can be specified by fixing the sensor to the same rigid body or disposing a third sensor that detects displacement.
(9) As shown in FIG. 11, the second detecting element 41a includes the magnetic sensor. The second detecting element 41a is disposed in at least one position of the tubular cavity 4 at which the positional relation to the tubular cavity 4 is specified, and detects the positional information of the inlet of the tubular cavity 4.

Consequently, for example, the coils 50a, 50b and 50c that perform position detection in accordance with the magnetic fields are disposed at the origin G, and generate the magnetic field signals to the second detecting element 41a. The coils 50a, 50b and 50c receive the generated magnetic field, and hence, the operator H who operates the tubular device 2 can obtain the absolute position of the object 3 based on the predetermined position without worrying about any wires or the like.

The second detecting element 41a is disposed at the position at which the positional relation between the position and the tubular cavity 4 is specified, and hence, it is possible to detect the positional information of the inlet of the tubular cavity 4.
(10) The endoscope apparatus includes the relative position detecting system of the tubular device to be inserted into the tubular cavity 4 of the object 3, and includes the shape sensor 10 that detects the shape of the tubular device 2, relative position detecting means for detecting the relative positional relation between at least one position of the first range in which the shape can be detected by the shape sensor 10 and at least one position on the tubular cavity 4, and the operating portion 7 that operates the shape of the inserting portion 5, so that it is possible to acquire the whole arrangement information of the tubular device 2 of the endoscope apparatus 1 to the tubular cavity 4.

In the endoscope apparatus 1 which performs the observation or treatment of the tubular cavity 4, the sampling of the lesion part, the foreign substances or the like in the tubular cavity 4, or the like, it is possible to detect the position or insertion state of the distal end of the tubular device in the tubular cavity 4. The inserting portion 5 can easily be inserted even into a region or an organ where the inserting portion is hard to be inserted. It is possible for the inserting portion 5 to rapidly reach a target place. The place to be observed can rapidly be specified, and hence, a great deal of time and labor to repeatedly insert the inserting portion are not required.

As to the disposing positions of the second detecting elements 41a, 41b, the second detecting element is disposed in at least one point on the tubular cavity 4. Specifically, the second detecting element is attached and fixed to the vicinity of the inlet of the tubular cavity 4 or a region that moves integrally with the vicinity of the inlet, fixed thereto with a string, rubber or the like, or attached to clothes put on during inspection, a treatment bed to be utilized during the inspection, or a support member such as a pillow.

A difference between the position of the second detecting element 41a or 41b and the position of the tubular cavity 4 can be eliminated by recording a difference between the second detecting element 41a or 41b and the first detecting element 40a or 40b and utilizing the difference in correction, when the first detecting elements 40a, 40b are disposed at the inlet of the tubular cavity 4.

It is to be noted that the present invention is not limited to the above embodiment as it is, and constitutional elements can be deformed and embodied without departing from the gist of the present invention in an implementation stage. In addition, various inventions can be formed by appropriately combining the constitutional elements disclosed in the above embodiment. For example, several constitutional elements may be deleted from all the constitutional elements described in the embodiment. Furthermore, the constitutional elements of different embodiments may appropriately be combined.

## Claims

1. A relative position detecting system of a tubular device, **characterized by** comprising:
a shape sensor for detecting a shape of the tubular device to be inserted into a tubular cavity of an object; and
relative position detecting means for detecting a relative positional relation between at least one position in a first range in which the shape of the tubular device is detectable by the shape sensor and at least one position in the tubular cavity.

2. The relative position detecting system of the tubular device according to claim 1, **characterized in that**
the tubular device includes an inserting portion having a flexibility to be inserted into the tubular cavity, and
the relative position detecting means detects the relative positional relation between at least one position in one of the first range and a second range in which a relative position to a range of the inserting portion is specifiable and at least one position in the tubular cavity.

3. The relative position detecting system of the tubular device according to claim 2, **characterized in that** the relative position detecting means acquires arrangement information of the tubular device to the tubular cavity based on the shape of the tubular device which is detected by the shape sensor, at least one position of the inserting portion, and at least one position in the tubular cavity.

4. The relative position detecting system of the tubular device according to claim 3, **characterized in that** the arrangement information of the tubular device includes positional information of a distal end of the inserting portion in the tubular device.

5. The relative position detecting system according to claim 2 or 3, **characterized in that** the relative position detecting means includes at least a first detecting element that is present in at least one position of the inserting portion in one of the first range and the second range, and a second detecting element that is present at the object, and detects relative positions of the first detecting element and the second detecting element.

6. The relative position detecting system according to claim 5, **characterized in that** the first detecting element and the second detecting element are present in a common coordinate system, and include a first transmitter or a first receiver fixed and disposed and a second receiver or a second transmitter configured to detect a relative position to the first transmitter or the first receiver, respectively.

7. The relative position detecting system according to claim 6, **characterized in that** each of a signal to be transmitted and received between the first transmitter and the second receiver and a signal to be transmitted and received between the second transmitter and the first receiver includes a magnetic field, an electromagnetic wave, a sound wave or an ultrasonic wave.

8. The relative position detecting system according to claim 5, **characterized in that** the first detecting element includes a magnetic sensor that detects a position in a space including a plane between the position and a predetermined position, and is mounted in at least one position on the tubular device in one of the first range and the second range.

9. The relative position detecting system according to claim 8, **characterized in that** the predetermined position of the first detecting element is an inlet region of the tubular cavity or an inside of the tubular cavity to acquire information including an arrangement of the tubular device.

10. The relative position detecting system according to claim 5, **characterized in that** the first detecting element includes one or both of an acceleration sensor that detects an acceleration to be applied to the tubular device and a rotation sensor that detects at least a rotation amount of the tubular device, and is mounted in at least one position on the tubular device in one of the first range and the second range.

11. The relative position detecting system according to claim 5, **characterized in that** the first detecting element is disposed at an insertion end of the inserting portion in the tubular device.

12. The relative position detecting system according to claim 5, **characterized in that** the second detecting element includes a magnetic sensor, and is disposed in at least one position in the tubular cavity.

13. The relative position detecting system according to claim 12, **characterized in that** the second detecting element is disposed at a position at which a positional relation between the position and the tubular cavity is specified, and detects positional information of an inlet of the tubular cavity.

14. The relative position detecting system according to claim 1 or 2, **characterized in that** the shape sensor includes an optical fiber sensor.

15. The relative position detecting system according to claim 14, **characterized in that** the shape sensor is disposed to the tubular device so that the shape of the tubular device is transmitted to the shape sensor using at least the inserting portion as a sensitive material.

16. The relative position detecting system according to claim 14 or 15, **characterized in that** the shape sensor detects bend information of the inserting portion in the tubular device.

17. An endoscope apparatus, **characterized by** comprising:
a shape sensor for detecting a shape of the tubular device to be inserted into a tubular cavity of an object;
relative position detecting means for detecting a relative positional relation between at least one position in a first range in which the shape of the tubular device is detectable by the shape sensor and at least one position in the tubular cavity; and
an operating portion that operates a shape of the inserting portion.

18. The endoscope apparatus according to claim 17, **characterized in that**
the tubular device includes an inserting portion having a flexibility to be inserted into the tubular cavity of the object, and
the relative position detecting means detects the relative positional relation between at least one position of one of the first range and a second range in which a relative position to a range of the inserting portion is specifiable and at least one position on the tubular cavity.

19. The endoscope apparatus according to claim 18, **characterized in that** the relative position detecting means acquires arrangement information of the tubular device to the tubular cavity based on the shape of the tubular device which is detected by the shape sensor, at least one position of the inserting portion, and at least one position on the tubular cavity.

20. The endoscope apparatus according to claim 19, **characterized in that** the arrangement information of the tubular device includes positional information of a distal end of the inserting portion in the tubular device.

21. The endoscope apparatus according to claim 18 or 19, **characterized in that** the relative position detecting means includes at least a first detecting element that is present in at least one position of the inserting portion in one of the first range and the second range, and a second detecting element that is present at the object, and detects relative positions of the first detecting element and the second detecting element.

22. The endoscope apparatus according to claim 21, **characterized in that** the first detecting element and the second detecting element are present in a common coordinate system, and include a first transmitter or a first receiver fixed and disposed and a second receiver or a second transmitter configured to detect a relative position to the first transmitter or the first receiver, respectively.

23. The endoscope apparatus according to claim 22, **characterized in that** each of a signal to be transmitted and received between the first transmitter and the second receiver and a signal to be transmitted and received between the second transmitter and the second receiver includes a magnetic field, an electromagnetic wave, a sound wave or an ultrasonic wave.

24. The endoscope apparatus according to claim 21, **characterized in that** the first detecting element includes a magnetic sensor that detects a position in a space including a plane between the position and a predetermined position, and is mounted in at least one position on the tubular device in one of the first range and the second range.

25. The endoscope apparatus according to claim 24, **characterized in that** the predetermined position of the first detecting element is an inlet region of the tubular cavity or an inside of the tubular cavity to acquire information including an arrangement of the tubular device.

26. The endoscope apparatus according to claim 21, **characterized in that** the first detecting element includes one or both of an acceleration sensor that detects an acceleration to be applied to the tubular device and a rotation sensor that detects at least a rotation amount of the tubular device, and is mounted in at least one position on the tubular device in one of the first range and the second range.

27. The endoscope apparatus according to claim 21, **characterized in that** the first detecting element is disposed at an insertion end of the inserting portion in the tubular device.

28. The endoscope apparatus according to claim 21, **characterized in that** the second detecting element includes a magnetic sensor, and is disposed in at least one position in the tubular cavity.

29. The endoscope apparatus according to claim 28, **characterized in that** the second detecting element is disposed at a position at which a positional relation between the position and the tubular cavity is specified, and detects positional information of an inlet of the tubular cavity.

30. The endoscope apparatus according to claim 17 or 18, **characterized in that** the shape sensor includes an optical fiber sensor.

31. The endoscope apparatus according to claim 30, **characterized in that** the shape sensor is disposed to the tubular device so that the shape of the tubular device is transmitted to the shape sensor using at least the inserting portion as a sensitive material.

32. The endoscope apparatus according to claim 30 or 31, **characterized in that** the shape sensor detects bend information of the inserting portion in the tubular device.
